Europäisches Patentamt

European Patent Office

Office européen des brevets

(19)

(11) Veröffentlichungsnummer: **0 224 225**
A1

# (12) EUROPÄISCHE PATENTANMELDUNG

(21) Anmeldenummer: 86116214.7

(22) Anmeldetag: 22.11.86

(51) Int. Cl.⁴: **A 61 B 10/00**
**A 61 B 17/20**

(30) Priorität: 28.11.85 DE 3542027

(43) Veröffentlichungstag der Anmeldung:
03.06.87 Patentblatt 87/23

(84) Benannte Vertragsstaaten:
AT BE CH DE ES FR GB IT LI LU NL SE

(71) Anmelder: BEHRINGWERKE Aktiengesellschaft
Postfach 1140
D-3550 Marburg 1(DE)

(72) Erfinder: Zerban, Ralf, Dr.
Bettewiese 13
D-3550 Marburg(DE)

(74) Vertreter: Meyer-Dulheuer, Karl-Hermann, Dr.
HOECHST Aktiengesellschaft Zentrale Patentabteilung
Postfach 80 03 20
D-6230 Frankfurt/Main 80(DE)

(54) Vorrichtung zum intradermalen Applizieren von Testsubstanzen und Impfstoffen.

(57) Bei der Vorrichtung zum intradermalen Applizieren von Testsubstanzen und Impfstoffen, ist ein mit einer Anzahl von Gruppen (8) von Spitzen (4) versehener Träger (3) auf der Mantelfläche eines um eine Achse (2) drehbar gelagerten kreisförmigen Zylinders (1) angeordnet.

FIG.1

BEHRINGWERKE AKTIENGESELLSCHAFT    HOE 85/B 022    DPh.HS/sch

Vorrichtung zum intradermalen Applizieren von Testsubstanzen und Impfstoffen

Die Erfindung betrifft eine Vorrichtung zum intradermalen Applizieren von Testsubstanzen und Impfstoffen, die eine Anzahl von Gruppen von dünnen Spitzen aufweist.

Eine Vorrichtung der genannten Art ist aus der DE-A-31 02 857 bekannt. Bei dieser Vorrichtung in Form eines achtbeinigen Stempels, bei dem die Gruppen von Spitzen in einer Ebene angeordnet sind, muß vor Gebrauch die Schutzkappe einer jeden Gruppe einzeln entfernt werden. Trotz erhöhten Anpreßdruckes ist es nicht möglich, die Haut gleichmäßig zu skarifizieren, was für die Dosierung der Testsubstanzen oder Impfstoffe wichtig ist.

Hier will die Erfindung Abhilfe schaffen. Die Erfindung, wie sie in den Ansprüchen gekennzeichnet ist, löst die Aufgabe dadurch, daß ein mit einer Anzahl von Gruppen von dünnen Spitzen versehener Träger auf der Mantelfläche eines um eine Achse drehbar gelagerten kreisförmigen Zylinders angeordnet ist.

Zum Schutz der Spitzen kann auf dem Träger eine mit Näpfen versehene Abdeckung angeordnet sein. In den Näpfen können Kissen aus saugfähigem Material angeordet sein. Der Zylinder kann eine Einrichtung zum Arretieren der Achse aufweisen. Er kann als Halbrundprofil oder als Hohlzylinder mit einer Nabe zur Aufnahme der Achse ausgebildet sein. Der Hohlzylinder kann als Teilschale ausgebildet sein. Die Achse kann mit Griffschalen oder dergleichen versehen sein.

Die durch die Erfindung erreichten Vorteile sind im wesentlichen darin zu sehen, daß bei entsprechender Dimensionierung des Zylinders eine beliebige Anzahl von Gruppen von Spitzen auf der Zylinderoberfläche angeordnet werden können, ohne daß der Anpreßdruck geändert werden muß. Da eine oder zwei Gruppen gleichzeitig auf die Haut gedrückt werden, beträgt die Kraft zum Anpressen nur ein Bruchteil dessen, was zum Andrücken eines planaren Stempels erforderlich wäre (z.B. ein Viertel gegenüber dem planaren achtfach Stempel). Damit verbessert sich die Dosierung der Substanzen. Ferner paßt sich ein abrollender Zylinder trotz größerer Anzahl von Gruppen der Oberfläche der Hautpartien besser an als ein planarer Stempel.

Im folgenden wird die Erfindung anhand von lediglich einen Ausführungsweg darstellenden Zeichnungen näher erläutert. Es zeigen

Figur 1 eine teilweise aufgeschnittene Seitenansicht der Vorrichtung,

Figur 2 die Seitenansicht einer Halbrundprofilausführung von Figur 1,

Figur 3 eine Hohlzylinderausführung (Teilschale) in Seitenansicht und

Figur 4 den Schnitt IV/IV der Figur 3.

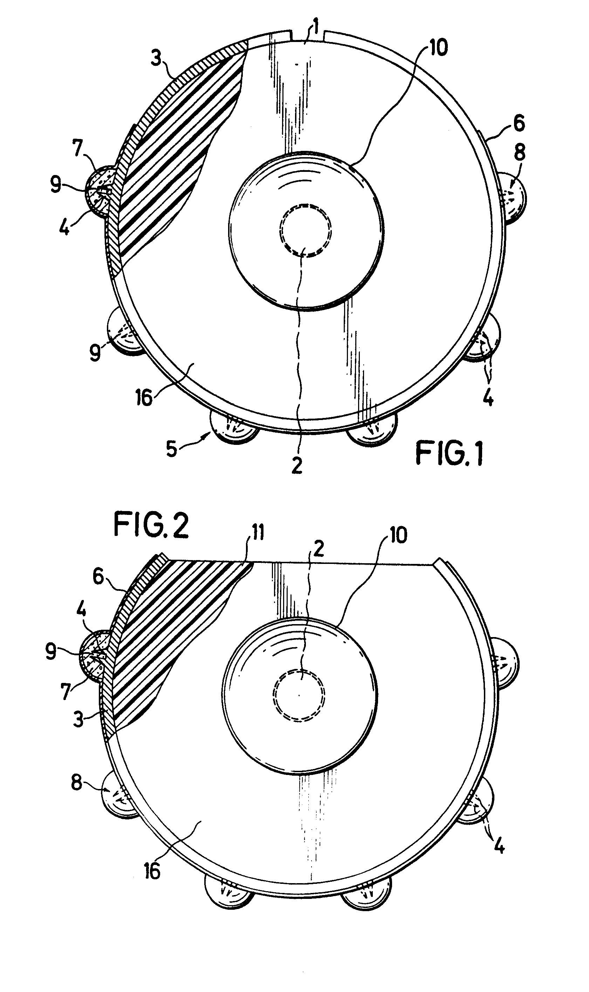

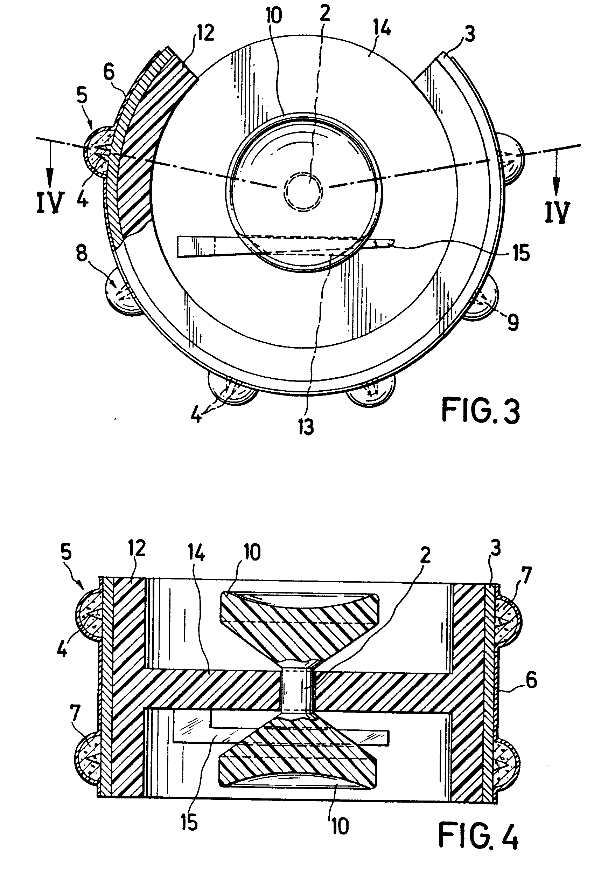

Nach der Ausführungsform gemäß Figur 1 besteht die Vorrichtung zum intradermalen Applizieren von Testsubstanzen und Impfstoffen aus einem Zylinder 1, der um eine Achse 2 drehbar gelagert ist. Auf der Mantelfläche des Zylinders 1 ist ein Träger 3 aus Metall oder Kunststoff angeordnet, der mit einer Anzahl von Gruppen 8 von Spitzen 4 versehen ist. Jede Gruppe 8 besteht aus wenigstens zwei Spitzen 4, die zweckmäßig so zueinander angeordnet sind, daß die Testsubstanz bzw. der Impfstoff durch Kapillarwirkung zwischen ihnen gehalten wird. Zum Schutz der Spitzen ist auf dem Träger 3 eine mit Näpfen 5 versehene Abdeckung 6 angeordnet. Die Näpfe 5 können zur Aufnahme der flüssigen Testsubstanzen oder Impfstoffen mit Kissen 7 aus vorzugsweise saugfähigem Material ausgekleidet sein. Als Abdeckung 6 kommen vorzugsweise tiefgezogene Kunststoffolien in Betracht, diese können entweder auf dem Träger 3 mit Kleber oder durch Heißversiegelung befestigt werden. Die Kissen 7 können in den Näpfen 5 befestigt sein, damit sie gemeinsam mit der Abdeckung 6 vom Träger 3 abgezogen werden können. Nach allgemeinen Erkenntnissen sollen die einzelnen Gruppen 8 untereinander einen Mindestabstand von 20 mm aufweisen. Die dargestellten Gruppen 8 bestehen aus je zwei Spitzen 4. Mehr als zwei Spitzen pro Gruppe sind ebenfalls möglich. Die Testsubstanz wird in dem von den beiden Spitzen 4 gebildeten Spalt 9 durch Kapillarwirkung gehalten. Die Gruppen 8 können ein- und/oder zweireihig auf dem Träger angeordnet sein. Zur Identifikation der einzelnen Gruppen können diese numeriert sein. Als Muster oder asymmetrisch auf dem Träger 3 angeordnet, wird die Zuordnung der skarifizierten Hautpartien zu den dazugehörigen Gruppen erleichtert. Zur besseren Handhabung kann die Achse 2 mit Griffschalen 10 versehen sein. Gemäß Figur 2 ist der Zylinder als Halbrundprofil 11 ausgebildet. Gemäß Figuren 3 und 4 besteht die erfindungsgemäße Vorrichtung aus einem Hohlzylinder bzw. aus einer Teilschale 12 eines Hohlzylinders. Hohlzylinder bzw. Teilschale 12 weisen eine Nabe 14 für die Achse 2 auf. Die Einrichtung 15, z.B. eine Feder, dient zum Arretieren der Achse 2. Die Einrichtung 15 kann an der Nabe 14 bzw. an der Stirnfläche 16 des Zylinders 1 befestigt sein und in eine Nut 13 in der Griffschale 10 oder dergleichen eingreifen. Die Testsubstanz bzw. der Impfstoff wird in üblicherweise auf die Spitzen aufgebracht.

Patentansprüche:

1. Vorrichtung zum intradermalen Applizieren von Testsubstanzen und Impfstoffen, dadurch gekennzeichnet, daß ein mit einer Anzahl von Gruppen (8) von Spitzen (4) versehener Träger (3) auf der Mantelfläche eines um eine Achse (2) drehbar gelagerten kreisförmigen Zylinders (1) angeordnet ist.

2. Vorrichtung nach Anspruch 1, dadurch gekennzeichnet, daß zwecks Schutz der Spitzen (4) auf dem Träger (3) eine mit Näpfen (5) versehene Abdeckung (6) angeordnet ist.

3. Vorrichtung nach Anspruch 2, dadurch gekennzeichnet, daß in den Näpfen (5) Kissen (7) aus saugfähigem Material angeordnet sind.

4. Vorrichtung nach Anspruch 1, dadurch gekennzeichnet, daß der Zylinder (1) eine Einrichtung (15) zum Arretieren der Achse (2) aufweise.

5. Vorrichtung nach Anspruch 1, dadurch gekennzeichnet, daß der Zylinder (1) als Halbrundprofil (11) ausgebildet ist.

6. Vorrichtung nach Anspruch 1, dadurch gekennzeichnet, daß der Zylinder (1) als Hohlzylinder mit einer Nabe (14) zur Aufnahme der Achse (2) ausgebildet ist.

7. Vorrichtung nach Anspruch 6, dadurch gekennzeichnet, daß der Hohlzylinder als Teilschale (12) ausgebildet ist.

8. Vorrichtung nach Anspruch 1, dadurch gekennzeichnet, daß die Achse (2) mit Griffschalen (10) versehen ist.

FIG.1

FIG.2

2/2

0224225

FIG.3

FIG.4

0224225

EUROPÄISCHER RECHERCHENBERICHT

Europäisches
Patentamt

Nummer der Anmeldung

EP 86 11 6214

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 117 247 (N. SHER) <br> * Figuren 2,3; Seite 1, Zeilen 69-78 * | 1,4,8 | A 61 B 10/00 <br> A 61 B 17/20 |
| Y |  | 2,3 | |
| | --- | | |
| X | FR-A-2 526 655 (C. LECLERC et al.) <br> * Figur * | 1 | |
| | --- | | |
| Y | EP-A-0 123 539 (J.C. BRENEMAN) <br> * Figuren 1-3 * | 2,3 | |
| | --- | | |
| A | WO-A-8 000 531 (L. BRENNAN) <br> * Figuren 1,4,6 * | 1 | |
| | ----- | | **RECHERCHIERTE SACHGEBIETE (Int. Cl.4)** |
| | | | A 61 B |

Der vorliegende Recherchenbericht wurde für alle Patentansprüche erstellt.

| Recherchenort | Abschlußdatum der Recherche | Prüfer |
|---|---|---|
| DEN HAAG | 06-03-1987 | ARGENTINI A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82